Europäisches Patentamt

**European Patent Office**

**Office européen des brevets**

(19)

(11) Numéro de publication : **0 048 446**
**B1**

(12)

# FASCICULE DE BREVET EUROPÉEN

(45) Date de publication du fascicule du brevet :
13.03.85

(21) Numéro de dépôt : 81107348.5

(22) Date de dépôt : 17.09.81

(51) Int. Cl.⁴ : **A 41 B 13/02**

(54) **Dispositif de fermeture perfectionné par adhésif, notamment pour des couches-culottes, et couches-culottes comportant un tel dispositif de fermeture.**

(30) Priorité : 23.09.80 FR 8020442

(43) Date de publication de la demande :
31.03.82 Bulletin 82/13

(45) Mention de la délivrance du brevet :
13.03.85 Bulletin 85/11

(84) Etats contractants désignés :
AT BE CH DE GB IT LI

(56) Documents cités :
FR-A- 2 078 794
FR-A- 2 259 553
US-A- 3 869 761
US-A- 4 010 754
US-A- 4 034 752
US-A- 4 209 016

(73) Titulaire : **BOUSSAC SAINT FRERES B.S.F. Société anonyme dite:**
**12, rue du Vieux Faubourg**
**F-59800 Lille (FR)**

(72) Inventeur : **Dussaud, Jacques**
**92, rue Jacquemars Giélée**
**F-59800 Lille (FR)**
Inventeur : **de Jonckheere, Raphael**
**797, Domaine de la Vigne**
**F-59910 Bondues (FR)**

(74) Mandataire : **Casalonga, Axel et al**
**BUREAU D.A. CASALONGA OFFICE JOSSE & PETIT**
**Baaderstrasse 12-14**
**D-8000 München 5 (DE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à un dispositif de fermeture par adhésif, notamment pour des couches-culottes à jeter pour enfants en bas âge, ainsi qu'à une couche-culotte à jeter comportant un tel dispositif de fermeture.

Des dispositifs de fermeture par adhésif pour des couches-culottes sont bien connus (voir par exemple brevets U.S. 3 180 335, 3 630 201, 4 047 530, 4 049 001, 4 050 463 et brevet français 74 36 169). Ces dispositifs de fermeture comprennent généralement une languette adhésive solidaire d'une partie de la couche-culotte et venant se fixer sur une autre partie de la couche-culotte en vue de la fermeture de cette dernière à la hauteur de la taille de l'enfant.

Les couches-culottes à jeter comprennent une enveloppe constituée par une mince feuille souple, imperméable portant sur une face un matelas absorbant. La languette adhésive est solidaire d'une partie de cette feuille, notamment de la partie formant l'arrière de la couche-culotte, et vient se fixer lors de la fermeture de la couche-culotte sur une autre partie de cette même feuille, notamment sur la partie formant l'avant de la couche-culotte.

Il arrive fréquemment que l'on veuille rouvrir et refermer la couche-culotte ainsi fermée, par exemple pour vérifier si l'enfant n'a pas encore mouillé le matelas absorbant ou pour rectifier la tenue de la couche-culotte, par exemple en vue de la resserrer à la taille. Or, il s'avère qu'une telle tentative d'ouverture sur une couche-culotte à jeter sur laquelle ladite feuille est extrêmement' mince provoque généralement, soit la déchirure de la languette adhésive par rapport à la partie arrière de la couche-culotte, soit l'arrachement de la zone de la feuille de la partie avant à laquelle adhère la languette adhésive, cette zone arrachée de la feuille restant collée sur la languette adhésive. Dans ce cas comme dans l'autre, il n'est plus possible de refermer la couche-culotte qui est ainsi inutilisable bien que le matelas absorbant puisse ne pas encore être mouillé.

Des dispositifs de fermeture analogue sont utilisés également pour d'autres applications, par exemple des enveloppes et emballages les plus divers en matériaux minces, et posent ici les mêmes problèmes, à savoir qu'une ouverture du dispositif provoque généralement une destruction définitive empêchant toute réutilisation qui pourrait néanmoins s'avérer intéressante dans certains cas.

La présente invention a pour objet un dispositif de fermeture par adhésif de ce type tel que défini dans la revendication 1, permettant, à la suite d'une première fermeture, au moins une ouverture suivie d'une seconde fermeture.

Le dispositif de fermeture conforme à l'invention comprend ainsi au moins deux languettes adhésives déchirables solidaires d'une partie d'une mince feuille formant enveloppe, ces deux languettes pouvant être fixées séparément par adhésif sur une autre partie de ladite feuille.

Il est ainsi possible d'utiliser l'une des deux languettes adhésives pour la première fermeture de l'enveloppe. Pour ouvrir de nouveau l'enveloppe, on déchire ladite languette ce qui n'entraîne aucun endommagement de l'enveloppe. Pour refermer ensuite l'enveloppe, on utilise la seconde languette adhésive qui peut se fixer sans problème sur ladite autre partie de l'enveloppe qui n'a pas été arrachée lors de l'ouverture préalable.

Suivant l'invention, les deux languettes adhésives sont constituées par une languette unique, de largeur accrue, solidaire d'une partie de l'enveloppe, et dont seule la partie destinée à la fixation sur l'autre partie de l'enveloppe est divisée en deux parties ou languettes placées côte à côte. L'avantage de ce mode de réalisation consiste dans le fait que la fabrication et la pose de ces languettes adhésives doubles à partir de ruban adhésif, peuvent être réalisées sur les machines déjà utilisées pour les languettes simples, la seule modification nécessaire consistant dans l'augmentation de la largeur du ruban adhésif dont sont formées les languettes et dans la prévision d'un poste de coupe des languettes sur une partie de leur longueur.

L'invention a également pour objet une couche-culotte à jeter, notamment pour enfants en bas âge, tel que définie dans la revendication 2, comportant ou ou deux dispositifs de fermeture réutilisables en cas de besoin.

En se référant au dessin annexé, on va décrire ci-après plus en détail un mode de réalisation illustratif et non limitatif d'un dispositif de fermeture conforme à l'invention, dans son application à une couche-culotte à jeter ; sur ce dessin :

la figure 1 est une vue en plan d'une couche-culotte à jeter équipée de deux dispositifs de fermeture conformes à l'invention, la couche-culotte étant représentée à plat ;

la figure 2 est une vue partielle de la couche-culotte de la figure 1, montrant un dispositif de fermeture après la première fermeture de la couche-culotte ;

la figure 3 est une vue correspondant à celle de la figure 2 montrant le dispositif de fermeture après la seconde fermeture de la couche-culotte.

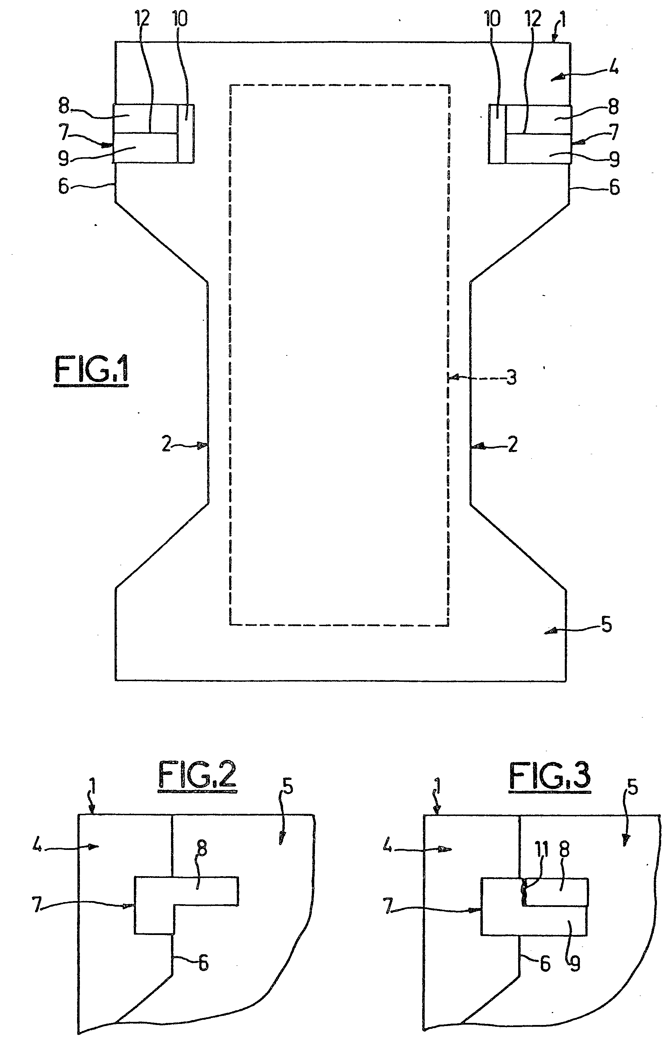

On reconnaît sur la figure 1 une couche-culotte pour enfants comprenant une enveloppe constituée par une mince feuille 1 souple, imperméable, par exemple en polyéthylène. La feuille 1 de forme générale rectangulaire présente, dans la zone de l'entrejambe, deux échancrures latérales 2 pour le passage des jambes de l'enfant. La feuille 1 porte sur une face un matelas absorbant 3 qui s'étend de part et d'autre de la zone de l'entrejambe jusque sur les parties plus larges 4 et 5 de la feuille 1, formant respectivement l'arrière et l'avant de la couche-culotte. Un voile perméable non représenté, par exemple en non-tissé, recouvre la feuille 1 et le matelas 3.

Pour pouvoir fermer la couche-culotte au niveau de la taille de l'enfant, la partie arrière 4 de la feuille 1 présente sur chaque bord latéral 6 un dispositif de fermeture 7 par adhésif. Chaque dispositif de fermeture 7 comprend deux languettes adhésives 8, 9 juxtaposées, solidaires de la partie arrière 4 de la feuille 1. Les deux languettes 8, 9 sont rabattues, avant utilisation, sur la face de la feuille 1 portant le matelas absorbant 3. La feuille 1 présente à cet endroit un morceau 10 d'une matière en feuille à laquelle les languettes adhésives 8 et 9 rabattues n'adhèrent que modérément.

Après avoir posé l'enfant sur la couche-culotte ouverte, on applique la partie avant 5 de la feuille 1 sur le ventre de l'enfant et on rabat les deux côtés de la partie arrière 4 par dessus les deux côtés de la partie avant 5, comme représenté sur la fig. 2. On détache ensuite l'un des deux rabats, en l'occurrence le rabat 8, du morceau de feuille 10, et on l'applique sur la partie avant 5 de la couche-culotte, c'est-à-dire directement sur la feuille 1.

Si l'on veut ouvrir de nouveau la couche-culotte, par exemple pour vérifier si le matelas absorbant 3 est mouillé, on déchire la languette adhésive 8 entre la zone de collage de cette dernière sur la partie avant 5 et la zone de fixation de la languette 8 à la partie arrière 4. On reconnaît sur la fig. 3 que la ligne de déchirure 11 de la languette 8 passe à ras du bord latéral 6 de la partie arrière 4 de la couche-culotte.

Pour refermer ensuite la couche-culotte, on décolle l'autre languette adhésive 9 du morceau de feuille 10 et on l'applique sur la partie avant 5 de la couche-culotte, comme représenté sur la fig. 3.

Pour permettre la déchirure des languettes 8 et 9, ces dernières sont avantageusement constituées par un matériau offrant une résistance à la déchirure inférieure à la résistance à l'arrachement des languettes adhésives par rapport à la feuille 1 de la couche-culotte et inférieure à la résistance à la déchirure de cette même feuille 1. Les languettes adhésives 8, 9 peuvent être par exemple constituées par du papier ou un matériau frangible analogue ayant néanmoins une résistance suffisante à la déchirure pour pouvoir supporter les efforts résultants du serrage de la couche-culotte à la taille de l'enfant. Il est également possible de les réaliser en un matériau plus résistant et de les munir chacune d'une ligne de moindre résistance facilitant la déchirure, ou d'une amorce de déchirure.

Suivant un mode de réalisation particulièrement avantageux de l'invention, chaque dispositif de fermeture 7 est constitué par un morceau de ruban adhésif d'une largeur supérieure à celle des rubans adhésifs utilisés pour la fabrication des dispositifs de fermeture usuels à simple languette adhésive. Ce morceau de ruban adhésif est fixé de façon usuelle sur toute sa largeur à la partie arrière 4 de la couche-culotte et la partie dépassant le bord latéral 6 de la partie arrière 4 est ensuite fendue dans le sens de la longueur du morceau de ruban adhésif comme indiqué par la ligne 12 sur la fig. 1. Il suffit ainsi de régler les machines déjà existantes sur une plus grande largeur de ruban adhésif et de prévoir un poste de coupe pour diviser en deux la partie du morceau de ruban adhésif dépassant le bord latéral 6.

Il va de soi que de nombreuses modifications et variantes peuvent être apportées à l'objet de l'invention tel que décrit ci-dessus et représenté sur le dessin annexé, sans pour autant sortir du cadre de la présente invention. Ainsi, les languettes adhésives 8, 9, au lieu d'être rabattues sur le morceau de matière en feuille 10 avant leur utilisation, pourraient également être munies chacune d'une pellicule de protection susceptible d'être arrachée pour mettre à nue la matière adhésive des languettes. Enfin, il serait possible de prévoir plus de deux languettes adhésives sur chaque dispositif de fermeture.

**Revendications**

1. Dispositif de fermeture par adhésif pour des enveloppes minces, notamment pour des couches-culottes à jeter, destiné à être rendu solidaire d'une partie de l'enveloppe et comprenant deux languettes adhésives rabattues avant utilisation sur une portion d'adhérence faible de ladite enveloppe et pouvant être successivement fixées par adhésif, lors de la fermeture de l'enveloppe, sur une autre partie de ladite enveloppe, caractérisé par le fait qu'il est constitué par un morceau de ruban adhésif comprenant un tronçon longitudinal destiné à être fixé sur sa largeur à demeure sur une partie (4) de l'enveloppe et un tronçon longitudinal dépassant ladite partie (4) de l'enveloppe, divisé par au moins une ligne de séparation longitudinale (12) en deux languettes adhésives (8, 9) disposées côte à côte initialement rabattues, la première languette (8) étant destinée à être fixée par adhésif sur l'enveloppe alors que la deuxième languette est encore rabattue, la deuxième languette (9) étant destinée à être fixée par adhésif sur l'enveloppe après déchirure de la première languette (8) à ras de sa jonction avec le premier tronçon longitudinal.

2. Couche-culotte à jeter, notamment pour enfants en bas âge, comprenant une enveloppe ouverte constituée par une mince feuille souple imperméable portant sur une face un matelas absorbant, un voile perméable recouvrant la feuille souple précitée et le matelas et deux dispositifs de fermeture par adhésif, solidaires d'une partie de l'enveloppe, chaque dispositif de fermeture comprenant deux languettes adhésives rabattues avant utilisation sur une portion d'adhérence faible de ladite enveloppe et pouvant être successivement fixées par adhésif, lors de la fermeture de l'enveloppe, sur une autre partie de ladite enveloppe, caractérisée par le fait que le dispositif de fermeture est constitué par un morceau de ruban adhésif comprenant un tronçon longitudinal fixé sur toute sa largeur à demeure sur une partie (4) de l'enveloppe et un tronçon longitudinal dépassant ladite partie (4) de l'enveloppe, divisé par au moins une ligne de séparation longitudinale (12) en deux languettes adhésives (8, 9) disposées côte à côte initialement rabattues, la première languette (8) étant destinée à être fixée par adhésif sur l'enveloppe alors que la deuxième languette est encore rabattue, la deuxième languette (9) étant destinée à être fixée par adhésif sur l'enveloppe après déchirure de la première languette (8) à ras de sa jonction avec le premier tronçon longitudinal.

3. Couche-culotte selon la revendication 2, caractérisée par le fait que l'enveloppe présente un morceau (10) d'une matière à laquelle les languettes adhésives (8, 9) rabattues n'adhèrent que modérément.

4. Couche-culotte selon la revendication 2, caractérisée par le fait que chacune des languettes adhésives est munie d'une pellicule de protection susceptible d'être arrachée pour mettre à nu la matière adhésive desdites languettes.

5. Couche-culotte selon l'une quelconque des revendications 2 à 4, caractérisée par le fait que chaque languette adhésive est munie d'une ligne de moindre résistance ou d'une amorce de déchirure permettant de définir une ligne de déchirure (11) passant à ras du bord latéral de la partie arrière de la couche-culotte.

## Claims

1. Device for closing by means of adhesive for thin covers, particularly for disposal nappy-pants, intended to be integrally attached to a part of the cover and incorporating two adhesive tabs folded back before use over a weakly adhesive portion of the said cover and capable of being successively fixed by means of adhesive, when the cover is closed, on another part of the said cover, characterised in that it consists of a piece of adhesive tape comprising a longitudinal section intended to be fixed over its width permanently on a part (4) of the cover and a longitudinal section extending beyond the said part (4) of the cover, divided by at least one longitudinal separation line (12) into two adhesive tabs (8, 9) arranged side by side and initially folded back, the first tab (8) being intended to be fixed by means of adhesive to the cover while the second tab is still folded back, the second tab (9) being intended to be fixed by means of adhesive to the cover after tearing of the first tab (8) level with its junction with the first longitudinal section.

2. Disposable nappy-pants, particularly for young children, incorporating an open cover consisting of a liquid-proof supple thin sheet bearing an absorbant pad on one face, a permeable film covering the abovementioned supple sheet and the pad and two devices for closing by means of adhesive, which are integrally fixed to a part of the cover, each closing device incorporating two adhesive tabs folded back before use on a weakly adhesive portion of the said cover and capable of being successively fixed by means of adhesive, when the cover is closed, to another part of the said cover, characterised in that the closing device consists of a piece of adhesive tape incorporating a longitudinal section fixed over ist whole width permanently to a part (4) of the cover and a longitudinal section extending beyong the said part (4) of the cover, divided by at least one longitudinal separation line (12) into two adhesive tabs (8, 9) arranged side by side and initially folded back, the first tab (8) being intended to be fixed by means of adhesive to the cover while the second tab is still folded back, the second tab (9) being intended to be fixed by means of adhesive to the cover after tearing of the first tab (8) level with its junction with the first longitudinal section.

3. Nappy-pants according to claim 2, characterised in that the cover has a piece (10) of a substance to which the folded-back adhesive tabs (8, 9) adhere only moderately.

4. Nappy-pants according to claim 2, characterised in that each of the adhesive tabs is equipped with a protective film capable of being torn to expose the adhesive substance of the said tabs.

5. Nappy-pants according to any one of claims 2 to 4, characterised in that each adhesive tab is equipped with a line of least resistance or a tearing-initiator making it possible to define a tearing line (11) passing level with the lateral edge of the rear part of the nappy-pants.

## Ansprüche

1. Klebeverschluß für dünne Hüllen, insbesondere für Wegwerfwindeln, der dazu bestimmt ist, mit einem Teil der Hülle verbunden zu sein und der zwei Klebezungen aufweist, die vor der Verwendung auf einen Bereich verminderter Haftung der Hülle umgeschlagen sind und die nacheinander beim Verschließen der Hülle auf einem anderen Bereich dieser Hülle festgeklebt werden können, dadurch gekennzeichnet, daß er durch ein Klebebandstück gebildet ist, das einen Längsabschnitt besitzt, der dazu bestimmt ist, auf seiner Breite ständig auf einem Teil (4) der Hülle befestigt zu sein und einen diesen Teil (4) der Hülle überragenden Abschnitt aufweist, der durch wenigstens eine Längstrennlinie (12) in zwei Klebezungen (8, 9) unterteilt ist, die Seite an Seite angeordnet zunächst umgeschlagen sind, wobei die erste Zunge (8) dazu bestimmt ist, auf der Hülle festgeklebt zu werden, während die zweite Zunge noch umgeschlagen ist und wobei die zweite Zunge (9) dazu bestimmt ist, auf der Hülle nach dem Abreißen der ersten Zunge (8) in der Höhe ihrer Verbindung mit dem ersten Längsabschnitt festgeklebt zu werden.

2. Wegwerfwindel, insbesondere für Kleinkinder, mit einer offenen Hülle, die durch eine dünne, undurchlässige Folie gebildet ist, die auf einer Seite einen saugfähigen Polster trägt, einem die vorgenannte weiche Folie und den Polster abdeckenden Tuch und mit zwei Klebeverschlüssen, die mit einem Teil der Hülle verbunden sind, wobei jeder Klebeverschluß zwei vor der Verwendung auf einen Bereich verringerter Haftung der erwähnten Hülle umgeschlagene Zungen aufweist, die nacheinander beim Verschließen der Hülle auf einem anderen Teil der erwähnten Hülle festgeklebt werden können, dadurch gekennzeichnet, daß der Verschluß durch ein Klebebandstück gebildet ist, das einen Längsabschnitt, der über seine gesamte Breite ständig auf einem Teil (4) der Hülle befestigt ist und einen Längsabschnitt aufweist, der den erwähnten Teil (4) der Hülle überragt und der durch wenigstens eine Längstrennlinie (12) in zwei Klebezungen (8, 9) unterteilt ist, die anfänglich umgeschlagen, Seite an Seite angeordnet sind, wobei die erste Zunge (8) dazu bestimmt ist, auf dem Umschlag festgeklebt zu werden, während die zweite Zunge noch umgeschlagen ist und wobei die zweite Zunge (9) dazu bestimmt ist, auf der Hülle nach dem Abreißen der ersten Zunge (8) in der Höhe ihrer Verbindung mit dem ersten Längsabschnitt befestigt zu werden.

3. Windel nach Anspruch 2, dadurch gekennzeichnet, daß die Hülle ein Stück (10) aus einem Werkstück aufweist, auf dem die umgeschlagenen Klebezungen (8, 9) nur beschränkt haften.

4. Windel nach Anspruch 2, dadurch gekennzeichnet, daß jede der Klebezungen mit einem Schutzhäutchen ausgerüstet ist, das abgezogen werden kann, um den Klebstoff der erwähnten Zungen freizulegen.

5. Windel nach irgendeinem der Ansprüche 2 bis 4, dadurch gekennzeichnet, daß jede Klebezunge mit einer Linie verringerter Festigkeit oder einer Einrißstelle versehen ist, die es gestattet, eine Reißlinie (11), die in der Höhe des Seitenrandes des hinteren Teils der Windel verläuft, zu definieren.

FIG.1

FIG.2

FIG.3